# EUROPEAN PATENT APPLICATION

(11) **EP 2 174 672 A1**
(43) Date of publication of application: **14.04.2010**
(21) Application number: 09171576.3
(22) Date of filing: 29.09.2009
(51) Int. Cl.: A61L 11/00, A61L 2/26, A61L 2/07, A61B 19/02, B65B 7/28, B65B 51/10, B65D 77/20, B65F 1/16, B65F 7/00

(54) **Container for collecting and transporting special materials, and method for processing such materials**

(30) Priority: 06.10.2008 NL 2002059
(71) Applicant: Ecama Holding B.V., 3862 WL Nijkerk (NL)
(72) Inventor: Versluis, Evert Cornelis Antonie, 3862 WL, Nijkerk (NL)
(74) Representative: de Vries, Johannes Hendrik Fokke

(57) **Abstract**

A container for collecting and transporting special materials, such as hospital waste and diagnostic samples, comprises a box (1) with a bottom (2), upright walls (3) and an opening (4) at its upper-side, said opening being surrounded by a closing edge (5). The container further comprises a cover (6) with a closing channel (7) at its circumference, a hot melt adhesive layer (8) being provided in the closing channel. The cover is adapted to be placed with its closing channel fittingly on the closing edge in such a manner that the closing edge is pressed into the adhesive layer. After pressing the closing edge (5) into the adhesive layer (8), the cover (6) is attached on the opening (4) by the adhesive strength of the adhesive layer only. The adhesive layer melts at a temperature of at least approximately 70°C, preferably at least 85°C. In a sterilisation process steam with a temperature of at least 121°C is supplied into an autoclave apparatus during such a period that the adhesive strength of the adhesive layer (8) is substantially lost and subsequently an underpressure is generated in the autoclave apparatus such that the cover (6) is released from the opening (4). Subsequently, steam with a temperature of at least 121°C is supplied again into the autoclave apparatus during a time period.

## Description

The invention relates to a container for collecting and transporting special materials, such as hospital waste and diagnostic samples, comprising a box with a bottom, upright walls and an opening at its upper side, said opening being surrounded by a closing edge, and a cover with a closing channel at its circumference, a hot melt adhesive layer being provided in the closing channel, wherein the cover is adapted to be placed with its closing channel fittingly on the closing edge in such a manner that the closing edge is pressed into the adhesive layer, and to a method for processing such materials.

A container of this type is known for example from EP-A-1 693 019. Although this container meets the requirements according to Dutch law regarding road transport of hazardous substances, the so-called packaging instruction P621 and meets the corresponding UN test criteria, this container is not suitable for processing the container with contents by means of a sterilisation process using steam with an increased temperature. For the application of such a sterilisation process it is important that the material within the container is well accessible for the steam during the sterilisation process. To this end several containers are known from the prior art, see for example EP-A-0 965 353, EP-A-1 010 399 and DE-C-19 730 472. In all known containers it is intended to establish an entrance opening automatically during the sterilisation process, by applying special means in the cover. The known containers show the disadvantage that applying these special means results in high manufacturing costs for the containers. Moreover the entrance opening obtained is small, so that the steam cannot penetrate well into the contents of the container. This is detrimental to the sterilisation process.

The invention aims to provide an improved container of this type, wherein the contents of the container can be made very well accessible during the application of the sterilisation process in a simple manner.

To this end the container according to the invention is characterized in that after pressing the closing edge into the adhesive layer, the cover is attached on the opening by the adhesive strength of the adhesive layer only and in that the adhesive layer melts at a temperature of at least approximately 70°C, preferably at least 85°C, wherein the adhesive strength of the adhesive layer (8) is substantially completely lost at a temperature of at least 110°C, preferably at least 120°C.

In this manner a container is obtained, wherein the adhesive layer which acts as the only connecting means fixing the cover on the opening, will give way with certainty due to the high temperature used in the sterilisation process, so that the cover will automatically come loose from the opening. Thereby the complete opening is available for the steam to penetrate into the materials contained in the container. In this manner the sterilisation process is very efficient, wherein a sterilisation of the materials or substances within the container is guaranteed. The invention is based on the insight that by avoiding mechanical connecting means between box and cover and choosing a suitable adhesive melting at a relatively low temperature, no special means are required anymore to create an entrance opening during the sterilisation process, whereas during normal use the UN test criteria of packaging instruction P621 are nevertheless met.

The invention also provides a method for processing special materials, such as hospital waste and diagnostic samples, wherein the material is collected and transported in containers according to anyone of the preceding claims, wherein the containers in closed condition are placed in an autoclave apparatus and are subjected to a sterilisation process, wherein steam with a temperature of at least 121°C is supplied into the autoclave apparatus during such a period that the adhesive strength of the adhesive layer is substantially lost and subsequently an underpressure is generated in the autoclave apparatus such that the cover is released from the opening, where after steam with a temperature of at least 121°C is supplied again into the autoclave apparatus during a time period.

The invention will be further explained by reference to the drawings showing very schematically an embodiment of the container according to the invention.

Fig. 1 shows a perspective view of an embodiment of the container according to the invention, wherein the cover is shown separated from the box.

Fig. 2 shows a cross-section of a part of the container of Fig. 1 with the cover placed on the box.

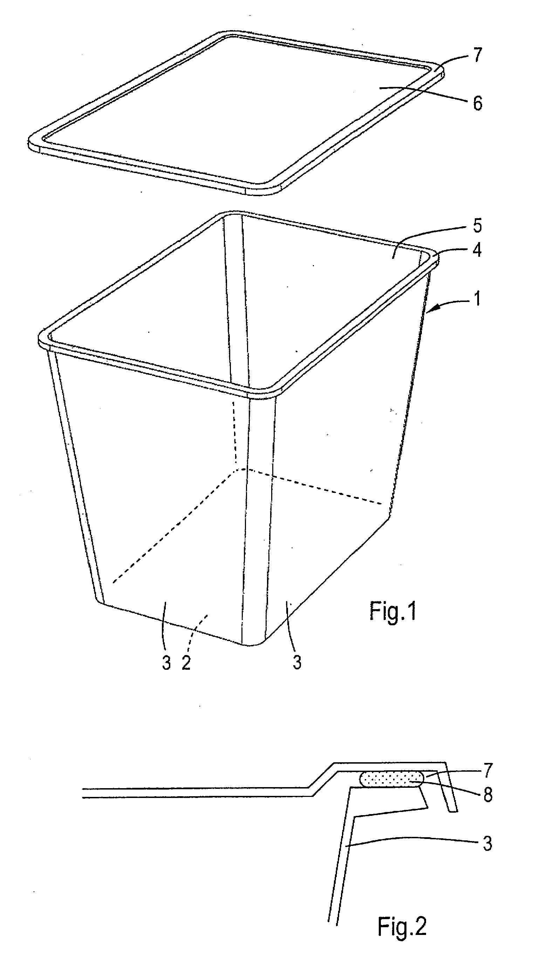

Fig. 1 shows a perspective view of a container for collecting and transporting special materials or substances, such as hospital waste and diagnostic samples. The container comprises a box or container 1 with a bottom 2 and upright sidewalls 3. The upright sidewalls 3 have an upper edge or closing edge 4 surrounding an opening 5, through which opening the box 1 can be loaded. A cover 6 is shown separated from the box 1, which cover 6 comprises at its circumference a closing channel 7. This closing channel 7 is provided with an adhesive layer 8 as shown in the cross-section of Fig. 2. During use the box 1 is loaded or filled up with for example hospital waste, wherein the cover 6 is each time removed from the box and thereafter located with the closing channel 7 on the closing edge 4 in a loose manner. When the box 1 has to be closed, the cover 6 is placed on the closing edge 4 and the closing edge 4 is pressed into the adhesive layer 8. In this manner the opening 5 is sealingly closed, wherein the cover 6 is fixed on the opening 5 by the adhesive strength of the adhesive layer 8 only.

This adhesive layer 8 is a so-called hot melt adhesive layer or melting adhesive layer, which comprises a thermoplastic material. The adhesive layer has such a composition that it shows good adhesive properties in a temperature range of approximately -20°C up to at least approximately 70°C, preferably at least 85°C. In this manner the containers described are very suitable for use in a method for processing special materials, such as hospital waste and diagnostic samples, wherein the closed containers are placed in an autoclave and are subjected to a sterilisation process. During the sterilisation process steam with a temperature of at least 121°C is supplied into the autoclave. As the adhesive layer melts already at a temperature of for example 85°C, the adhesive will drip out of the closing channel and after being exposed to the steam temperature during a time period, the adhesive strength will be substantially completely lost. Subsequently an underpressure or vacuum is generated within the autoclave, whereby the cover 6 will be removed automatically from the box 1 and the opening 5 is completely released. During a next supply of steam with a temperature of 121°C or higher, the steam can penetrate deeply into the materials within the box 1, whereby a very good sterilisation of these materials is guaranteed. The sterilisation process may comprise two, three or more cycles of supplying steam and generating an underpressure. One cycle may take for example 7-10 minutes. After filling the autoclave the process may comprise a preheating phase of approximately 10 minutes in a usual manner, wherein the autoclave is raised in temperature by means of steam under pressure. Following the cycles of generating a vacuum and supplying steam, a period of for example 30 minutes follows, during which the autoclave is maintained at the temperature of at least 121°C.

Experiments have shown very good results with a so-called hot melt foam adhesive layer, i.e. an adhesive layer foamed with air. Preferably the ratio adhesive/air of this foam adhesive layer is at least 1:3, more preferably at least 1:4. In experiments with a hot melt foam adhesive layer, wherein a volume of foam adhesive of 5,5 cm³ was formed out of 1 cm³ adhesive, an excellent adhesive strength in the temperature range of -20°C up to 85°C was obtained on the one hand and on the other hand the adhesive strength was removed with certainty such that the cover was released from the box in the sterilisation process described without any problems. The box 1 closed with the cover 6 did meet UN test criteria of the packaging instruction P621. The adhesive layer can be provided in the closing channel by means of an adhesive nozzle with a very small exit opening, wherein a very high air pressure is used. To obtain an adhesive layer with sufficient width a nozzle with a dual exit opening can be used.

It is noted that the box and cover can be made of any suitable material, for example a plastic material or waterproof cardboard. If desired, a separate small opening with closing edge can be provided in the cover 6, which small opening can be closed by means of a second cover with closing channel. Also this second cover can be fixed to the opening after final closing of the opening by the adhesive strength of the adhesive layer only. The sterilisation process may take place at a central location, for example at a waste processing company or locally, for example at a hospital.

De invention is not restricted to the above-described embodiment which can be varied in a number of ways within the scope of the claims.

## Claims

1. A container for collecting and transporting special materials, such as hospital waste and diagnostic samples, comprising a box (1) with a bottom (2), upright walls (3) and an opening (4) at its upper-side, said opening being surrounded by a closing edge (5), and a cover (6) with a closing channel (7) at its circumference, a hot melt adhesive layer (8) being provided in the closing channel, wherein the cover is adapted to be placed with its closing channel fittingly on the closing edge in such a manner that the closing edge is pressed into the adhesive layer, **characterized in that** after pressing the closing edge (5) into the adhesive layer (8), the cover (6) is attached on the opening (4) by the adhesive strength of the adhesive layer only and **in that** the adhesive layer melts at a temperature of at least approximately 70°C, preferably at least 85°C, wherein the adhesive strength of the adhesive layer (8) is substantially completely lost at a temperature of at least 110°C, preferably at least 120°C.

2. Container according to claim 1, wherein the adhesive layer (8) maintains its adhesive strength in a temperature range of approximately -20°C up to at least approximately 70°C, preferably at least 85°C.

3. Container according to claim 1 or 2, wherein the adhesive layer (8) is a hot melt foam adhesive layer with a ratio of adhesive/air of at least 1:3, preferably at least 1:4.

4. Method for processing special materials, such as hospital waste and diagnostic samples, wherein the material is collected and transported in containers according to anyone of the preceding claims, wherein the containers in closed condition are placed in an autoclave apparatus and are subjected to a sterilisation process, wherein steam with a temperature of at least 121°C is supplied into the autoclave apparatus during such a period that the adhesive strength of the adhesive layer (8) is substantially lost and subsequently an underpressure is generated in the autoclave apparatus such that the cover (6) is released from the opening (4), whereafter steam with a temperature of at least 121°C is supplied again into the autoclave apparatus during a time period.

5. Method according to claim 4, wherein the sterilisation process comprises at least three cycles of supplying steam and generating an underpressure.
